# EUROPEAN PATENT SPECIFICATION

(11) **EP 1 210 950 B1**
(45) Date of publication and mention of the grant of the patent: **19.10.2005**
(21) Application number: 00850205.6
(22) Date of filing: 04.12.2000
(51) Int. Cl.: A61K 45/06, A61K 31/505, A61P 33/02, A61K 31/44, A61K 31/70

(54) **Composition against Coccidiosis**
Zusammensetzung gegen Coccidiose
Composition contre la coccidiose

(43) Date of publication of application: 05.06.2002
(73) Proprietor: New Pharma Research Sweden AB, 223 70 Lund (SE)
(72) Inventor: Abdul-Rahman, Shoaa, 224 76 Lund (SE)
(74) Representative: Bjerre, Nils Birger Johannes

(56) References cited:
- EP-A- 0 642 797
- WO-A-97/02021
- FR-A- 2 605 221
- GB-A- 1 140 315

## Description

The present invention relates to a composition for use as a medicament, and use of a composition for the manufacture of a medicament for treatment of coccidiosis.

Coccidiosis is a disease of the intestinal lining of poultry, for example chickens, caused by protozoan coccidial parasites of the genus Eimeria, such as Eimeria tenella, Eimeria necatrix, Eimeria acervulina, Eimeria brunetti, Eimeria maxima, Eimeria mitis, Eimeria mivati, Eimeria praecox and Eimeria hagani. Species belonging to said genus cause clinical disease in chickens and immunity to any one species does not protect birds against infection with other species. Coccidiosis is seen most commonly in chickens between 3 and 6 weeks of age. Coccidiosis is caused by protozoa of the family Eimeriidae. Various sites in the intestine are infected. The infectious process is rapid (4-7 days) and is characterised by parasite replication in host cells with extensive damage to the intestinal mucosa. Clinical disease occurs only after ingestion of relatively large numbers of sporulated oocysts by susceptible birds. Both clinically infected and recovered birds shed oocysts in their droppings, which contaminate feed, dust, water, litter and soil.

Anticoccidial drugs are available on the market for prevention and treatment of coccidiosis in especially chickens and turkeys. Anticoccidials are generally given to poultry in the feed to prevent acute disease. Water medication is generally preferred over feed medication for treatment. Antibiotics are sometimes used to improve rate of recovery and prevent secondary infections.

Among anticoccidial drugs previously known, the following may be mentioned: Amprolium, Clopidol, folic acid antagonists, halofuginone hydrobromide, ionophores (monensin, salinomycin, lasalocid, narasin, maduramicin), Nicarbazin, nitrobenzamides (e.g. dinitolmide), quinolones (e.g. buquinolat, decoquinate, nequinate) and Robenidine. Due to the widespread resistance to most anticoccidial drugs, there is a continuous need for new compositions having fewer side effects.

FR-A-2 605 221 relates to compositions comprising monensine lactone against coccidiosis and that in combination with aminoglycoside antibiotics the activity of monensine is increased.

GB-A-1 140 315 relates to a composition for accelerating the growth rate of animals containing a tetracycline antibiotic, neomycin and penicillin, and showing the synergistic action of said three agents against coccidiosis.

According to one aspect of the invention there is provided a composition for use as a medicament comprising a three-component mixture of folic acid, aminoglycosides and antiulcerative agents, said components being present in therapeutically effective amounts, optionally in admixture with therapeutically acceptable carriers.

According to another aspect of the invention use of a composition for the manufacture of a medicament for treatment of coccidiosis is provided, said composition comprising a mixture of at least two components selected from the group consisting of folic acid, aminoglycosides and antiulcerative agents.

The composition of the invention contains three major components, viz. (A) folic acid, (B) aminoglycosides and (C) antiulcerative agents. The composition may comprise a mixture of two components optionally selected among the three components mentioned above for the treatment of coccidiosis.

According to a preferred embodiment, the composition contains a complexing agent, for example EDTA (ethylenediaminetetraacetic acid).

An interesting and surprising feature of the invention is the fact that according to published literature the individual components are not effective in treating coccidiosis. A composition comprising at least two of the above components is however effective, which will appear from tests described below.

Each major component will now be described in detail below.

### Folic acid

Folic acid has the formula

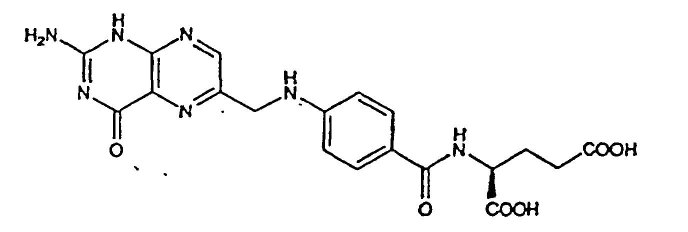

Folic acid is an important ingredient in essentially all foods and feeds. Beer and feed yeasts, especially, are rich in folic acid. Further, all green parts of plants, extracted soybean meal, potatoes, wheat bran and liver contain a relatively large amount of folic acid. Folic acid is in particular involved in the synthesis of amino acids and nucleic acids. Along with vitamin B₁₂, folic acid is essential for the formation of red and white blood cells.

Folic acid is used as a supplement to animal feed. Animals have a considerable synthesis of folic acid owing to the microorganisms in the gastrointestinal tract. Especially in poultry and in young animals the initial amount of folic acid available in this way is inadequate and supplements to the feed are necessary. The dosage generally recommended for feed supplements in case of chickens, pullets, broilers, hens and turkeys is about 0.6 - 1.5 mg per kg of feed (88% dry matter). Concerning the use of folic acid as a feed additive reference is made to BASF Fine Chemicals. Know-how and quality for animal nutrition, "On feed additives: Technical Information". Edition 97/98, p. 33, 46-51. It should be mentioned that folic acid has no anticoccidial effect in poultry.

### Aminoglycosides

Aminoglycosides are used primarily in the treatment of infections caused by aerobic gram-negative organisms. They are not active against anaerobic organisms. Aminoglycosides are divided into three classes: narrow-spectrum aminoglycosides, broad-spectrum aminoglycosides and miscellaneous aminoglycosides. As examples of aminoglycoside the following are mentioned.

Streptomycin was the earliest aminoglycoside introduced. It is active against mycobacteria, Leptospira, Francisella tularensis and Yersinia pestis. Dihydrostreptomycin is chemically very similar to streptomycin.

Neomycin became available for use a few years after streptomycin. Neomycin has been effective against many gram-negative organisms and Staphylococcus aureus. The use of neomycin is limited by a relatively high risk of toxicity.

Kanamycin was introduced as a less toxic alternative to older aminoglycosides and was soon followed by gentamicin and later by amikacin.

Amikacin was developed from kanamycin and has the broadest spectrum of activity of the aminoglycosides. It is considered effective against strains not susceptible to other aminoglycosides because it resists some aminoglycoside inactivating enzymes.

Apramycin is an aminocyclitol antibiotic with a chemical structure very similar to that of the aminoglycosides but different enough to leave it unaffected by many aminoglycoside inactivating enzymes. Apramycin is active against Staphylococcus aureus, many gram-negative organisms and some mycoplasma strains. Apramycin has been reported to be effective in vitro among others against Salmonella species which are resistant to streptomycin and neomycin.

Among those mentioned above, dehydrostreptomycin has been found to be highly preferred as component in the composition of the invention. As mentioned above, aminoglycosides are used in the treatment of infections caused by aerobic organisms and as such they are not active against parasites. However, according to the invention, a therapeutic effect against coccidiosis is shown when present in admixture with at least one of the remaining components, viz. component A and component C, which latter will be described in detail below.

### Antiulcerative agents

Antiulcerative agents can be divided into two groups, viz. proton pump inhibitors and histamine H₂-antagonists. Chemically, proton pump inhibitors (PPIs) consist of a substituted benzimidazole moiety linked by an essential sulfinylmethyl group to a substituted pyridine ring. Omeprazol is an important member of the above group and is the active ingredient in Losec®, a preparation developed by Astra, Sweden. Histamine H₂-antagonists are structural analogs of histamine that competitively and surmountably block the H₂-receptors located on the basolateral membrane of the parietal cell. Among members of the latter group, the following may be mentioned: Cimetidine, Nizatidine, Famotidine and Ranitidine. As to antiulcerative agents in general and their therapeutic effects especially reference is made to Ullmann's Encyclopedia of Industrial Chemistry, Sixth Edition, 2000 Electronic Release, ANTIULCER DRUGS.

The compositions according to the invention are quite generally suitable for protecting poultry, i.e. for treating useful farm poultry, such as chickens, turkeys, ducks or geese or also other birds, such as for example pheasants, etc.

The compositions according to the invention are used in the form of therapeutic preparations for treating animals, especially birds, which are attacked by coccidia. The expression "therapeutic" also includes prophylaxis. Such therapeutic preparations conventionally may contain therapeutically acceptable carriers. Thus, such preparations find application in broiler farms or poultry rearing houses for pullets where a high burden of infection is produced for the poultry population because of the permanent forms of the coccidia (oocysts) which are constantly being excreted in the droppings. The use of the composition according to the invention and methods for combating coccidiosis are carried out in the normal manner. In view of the localisation of the coccidia in the intestinal tract, an oral administration is primarily suitable. The compositions according to the invention may in this case be mixed with feed stuffs or with drinking water.

The concentrations of the compositions in feed stuffs or in drinking water may widely vary within certain limits. In general the concentrations are within the range of 5-5000 ppm (by weight) of the composition based on the feed stuff or drinking water. A preferable range is 50-2000 ppm. A most preferable range is 50-300 ppm. It is within the competence field of the veterinary to choose the concentration of each component in the final composition. Such concentration is herein often referred to "a therapeutically effective amount" and is defined as the amount which is sufficient for successfully combating coccidiosis keeping the side effects to a minimum. Various concentrations of the components will appear from the Examples below.

The concentrations of the compositions are based on the feed or drinking water preparations ad lib, i.e. for free feed or drinking water consumption during a normal practical fattening or rearing period. All feed formulations conventional in the poultry industry are suitable as carriers for the compositions according to the invention. The formulations specified below for poultry feed are examples of normal practical formulations. Further, it is also possible, however, to use other feedstuffs based on some types of cereal grains which contain vitamin concentrates, mineral concentrates or other active substances and feed additives in any concentration. Both conventional dry mealy or pelletised feedstuffs but also liquid feed suspensions including feedstuffs such as distillers' residues and milk by-products can be used in the case of the compositions according to the invention.

To prepare the poultry feed a concentrated premix is usually first made up which contains at least two of the components of the composition in high concentration, for example 0.2-75% by weight. For this purpose the components are either dispersed or mixed in inert carrier substances, such as vermiculite, diatomaceous earth, attapulgite, calcium carbonate, etc., together with physiologically harmless carriers, such as propylene glycols, polyethylene glycols, inert oils, such as vegetable oils, highly refined mineral oils, ethanol, water, or aqueous alcohols. Organic carrier material, such as wheat bran, shredded maize, soybean flour, lucerne meal, rice husks or ground maize cobs and any other organic carrier substances from vegetable products are likewise suitable for this purpose.

As mentioned above, the compositions according to the invention may further also be administered to poultry together with the drinking water. Their inclusion in the drinking water is achieved by adding a form of the composition concerned which is soluble in water or can be suspended in water to the drinking water in a suitable quantity. Such preparations are in general produced by selecting a water-soluble form of the composition. Water-insoluble forms, for example suspensions, may also be used. In such cases physiologically harmless auxiliary agents which keep the composition according to the invention in suspension in water over a prolonged period are used. Auxiliary agents which are suitable for this purpose are swelling agents, such as alginates, gelatine, carboxymethylcellulose or polyvinylpyrrolidone. Surfactant compounds, such as for example naphthalenesulfonates, alkylbenzenesulfonates, or polyoxyethylene sorbitan esters may be used for bringing the compositions into suspensions. Normally, a concentrated suspension or a dry formulation of the composition according to the invention and the suspension agents is produced in certain mixing ratios and is then diluted with the drinking water to the required application concentration or the premixes are mixed in suitable concentrations with the feedstuffs normal in practice. The drinking water or feed so medicated is then fed to the poultry either initially ad lib or for a certain period.

Although the invention has been described above with particular reference to the treatment of poultry against coccidia infections, treatment of other domestic and useful animals, for example other birds, rabbits, pigs and ruminants is also within the scope of the invention.

It is within the scope of the invention to use a mixture of anticoccidial drugs previously known and the composition of the invention in combating coccidiosis.

The invention is explained by the Examples below.

Table A below summarises examples of compositions of the invention. The compositions are formed by mixing the components in the amounts defined in the table in a conventional mixer.

**TABLE A**

| Example | Component A | amount ppm by weight | Component B | amount ppm by weight | Component C | amount ppm by weight | Complexing agent, ppm by weight |
|---|---|---|---|---|---|---|---|
| 1 | folic acid | 66.7 | dhsm*) | 66.7 | Famotidine | 66.7 | |
| 2 | -"- | 55 | -"- | 90 | Nizatidine | 55 | |
| 3 | - | - | -"- | 110 | -"- | 90 | |
| 4 | folic acid | 55 | -"- | 90 | Omeprazole | 55 | |
| 5 | - | - | -"- | 112 | -"- | 88 | |
| 6 | folic acid | 50 | -"- | 100 | Cimetedine | 50 | |
| 7 | - | - | -"- | 124 | -"- | 76 | |
| 8 | - | - | -"- | 116 | Ramitidine | 84 | |
| 9 | folic acid | 55 | -"- | 90 | -"- | 55 | |
| 10 | -"- | 100 | -"- | 100 | - | - | |
| 11 | - | - | -"- | 100 | Famotidine | 100 | |
| 12 | folic acid | 90 | Amikacin | 110 | - | - | |
| 13 | -"- | 40 | Apramycin | 160 | - | - | |
| 14 | -"- | 70 | Neomycin | 130 | - | - | |
| 15 | - | - | -"- | 145 | Famotidine | 55 | |
| 16 | folic acid | 140 | dhsm | 60 | - | - | |
| 17 | - | - | -"- | 75 | Famotidine | 125 | |
| 18 | folic acid | 66.7 | -"- | 66.7 | -"- | 66.7 | EDTA 50 |
| 19 | -"- | 30 | Neomycin | 120 | - | - | -"- 50 |
| 20 | -"- | 50 | -"- | 100 | - | - | -"- 50 |
| 21 | -"- | 100 | - | - | Famotidine | 100 | |
| 22 | -"- | 40 | Kanamycin | 160 | - | - | |

| | | | | | | | |
|---|---|---|---|---|---|---|---|
| *) dhsm = dihydrostreptomycin | | | | | | | |

### Examples of animal feed compositions

The following Examples relate to animal feed compositions which can be used for administering the composition according to the invention.
I. Fattening feed for broilers

| Composition in % by weight | |
|---|---|
| Fishmeal (60-65% | 6.0 |
| Feeding yeast | 2.0 |
| Beef dripping | 4.7 |
| Coarse soybean meal (44%) | 24.0 |
| Lucerne green meal | 1.0 |
| Maize | 44.89 |
| Wheat | 6.35 |
| Coarse wheat flour | 8.5 |
| Phosphate feed lime | 1.4 |
| Carbonate feed lime | 0.96 |
| Trace element premix* | 0.04 |
| Cattle salt | 0.09 |
| Methionine DL | 0.07 |
| | 100.00 |

(a) The following are added per kg of feed:

| Vitamin | | |
|---|---|---|
| A | I.U. | 12,000 |
| D₃ | I.U. | 1,500 |
| E | mg | 18 |
| B₁ | mg | 1.5 |
| B₂ | mg | 6 |
| Pantothenic acid | mg | 9 |
| Nicotinic acid | mg | 24 |
| Vitamin | | |
| B₆ | mg | 4.5 |
| B₁₂ | µg | 24 |
| K₃ | mg | 3 |
| Choline chloride | mg | 1,300 |

* (b) 1.0 kg of feed contain

| | | |
|---|---|---|
| Mn | mg | 106 |
| Zn | mg | 71 |
| Fe | mg | 44 |
| Cu | mg | 3.56 |
| I | mg | 0.4 |
| Co | mg | 0.16 |

II. Rearing feed for chickens (0-8 weeks)

| Composition in % by weight | |
|---|---|
| Fishmeal (60-65% | 5.0 |
| Lucerne green meal | 6.0 |
| Coarse soybean meal (44%) | 13.0 |
| Feeding yeast | 2.8 |
| Beef dripping | 2.0 |
| Barley | 6.0 |
| Oats | 6.0 |
| Maize | 37.0 |
| Wheat | 13.0 |
| Wheat bran | 7.3 |
| Carbonate feed lime | 1.29 |
| .RTM. Hostaphos | 0.57 |
| Trace element premix* | 0.04 |
| | 100.00 |

| | |
|---|---|
| * see Example I (b); furthermore, as described in Example I (a), vitamins are added | |

III. Rearing feed for chickens (9-20 weeks)

| Composition in % by weight | |
|---|---|
| Lucerne green meal | 6.0 |
| Coarse soybean meal (44%) | 10.24 |
| Feeding yeast | 1.8 |
| Beef dripping | 2.0 |
| Barley | 8.0 |
| Oats | 6.0 |
| Maize | 40.0 |
| Wheat | 15.0 |
| Wheat bran | 8.3 |
| Carbonate feed lime | 1.53 |
| .RTM. Hostaphos | 1.02 |
| Trace element premix* | 0.04 |
| Methionine DL | 0.07 |
| | 100.00 |

| | |
|---|---|
| * see Example I (b); furthermore, vitamins are added, see Example I (a), | |

IV. Rearing feed for chickens (from 21st week onwards)

| Composition in % by weight | |
|---|---|
| Fishmeal (60-65% | 1.5 |
| Coarse soybean meal (44%) | 19.31 |
| Beef dripping | 1.0 |
| Oats | 6.7 |
| Maize | 40.0 |
| Wheat | 18.0 |
| Coarse wheat flour | 3.0 |
| Methionine DL | 0.07 |
| Feed paprika | 0.3 |
| Carbonate feed lime | 8.16 |
| .RTM. Hostaphos | 1.91 |
| Trace element premix* | 0.05 |
| | 100.00 |
| Furthermore vitamins are added as described in Example I (a). | |

### Biological Examples

### A1

### Anticoccidiocidal activity

### Materials and Methods

### Chickens

A total of 92 one day old Habbared X breed chickens were used in the test. They were allocated into 4 groups (23 birds in each group), one group serving as a control group.

### Medication groups:

Each composition to be tested was mixed in a sufficient amount of feed for each group and was used for daily feeding from the day zero of the test. Fresh water was supplied ad-libetum.
The test groups were:

| | |
|---|---|
| Group No. 1 | feed containing 200 ppm of a combination of folic acid and dihydrostreptomycin in a ratio of 1:1, |
| Group No. 2 | feed containing 200 ppm of a combination of Famotidine and dihydrostreptomycin in a ratio of 1:1, |
| Group No. 3 | feed containing standard anticoccidial agent (Clopidol, 125 ppm), |
| Group No. 4 | infected non-treated group. |

The results appear from Table 1.

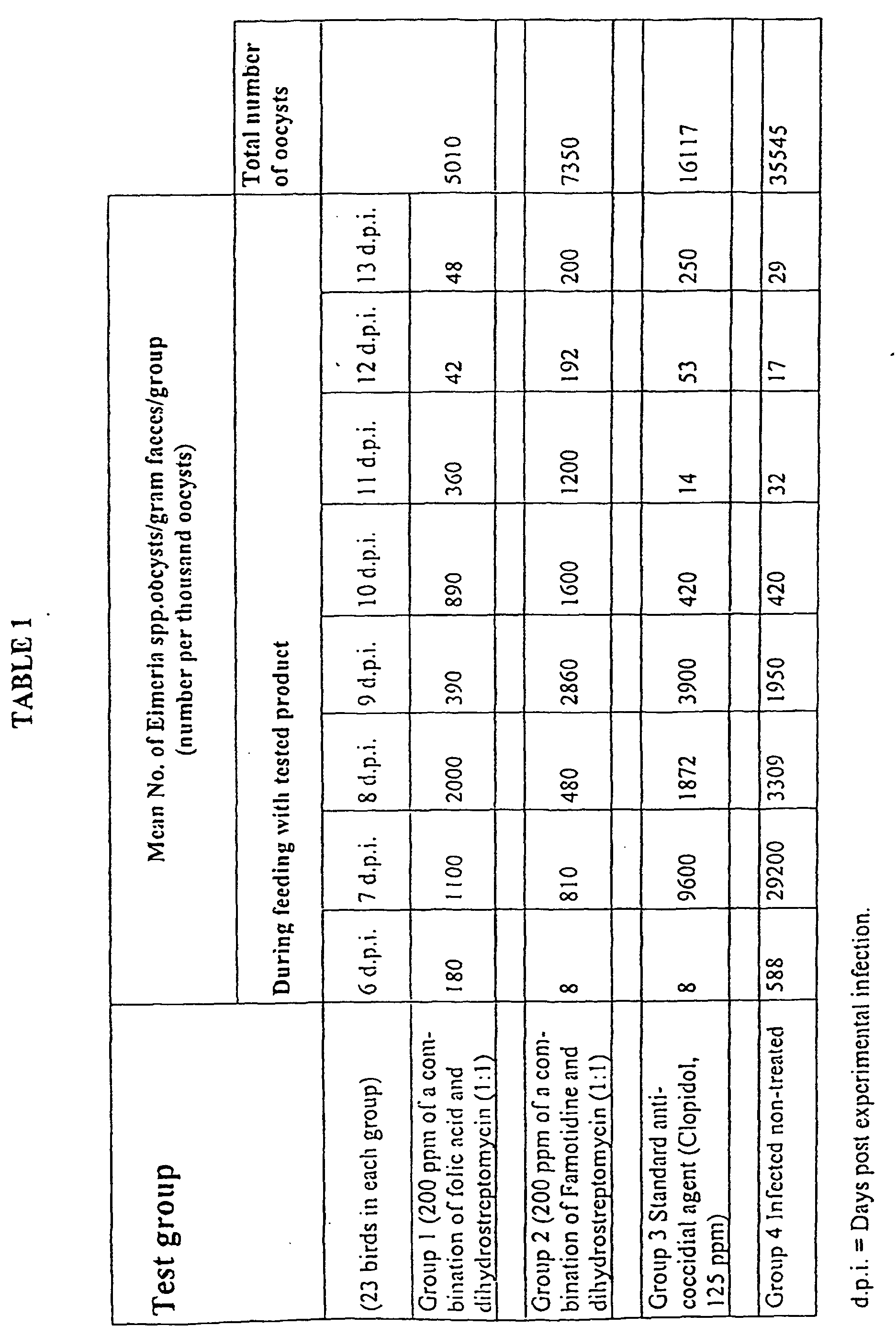

### A2

### Anticoccidiocidal activity

### Materials and Methods

### Chickens

A total of 88 one day old Habbared X breed chickens were used in the test. They were allocated into 4 groups (22 birds in each group), one group serving as a control group.

### Medication groups:

Each composition to be tested was mixed in a sufficient amount of feed for each group and was used for daily feeding from the day zero of the test. Fresh water was supplied ad-libetum.
The test groups were:

| | |
|---|---|
| Group No. 1 | feed containing 200 ppm of a combination of folic acid, Famotidine, dihydrostreptomycin (1:1:1), |
| Group No. 2 | feed containing 200 ppm of a combination of folic acid, Famotidine, dihydrostreptomycin (1:1:1) + EDTA (50 ppm), |
| Group No. 3 | Clopidol, 125 ppm |
| Group No. 4 | infected non-treated group. |

The results appear from Table 2 below.

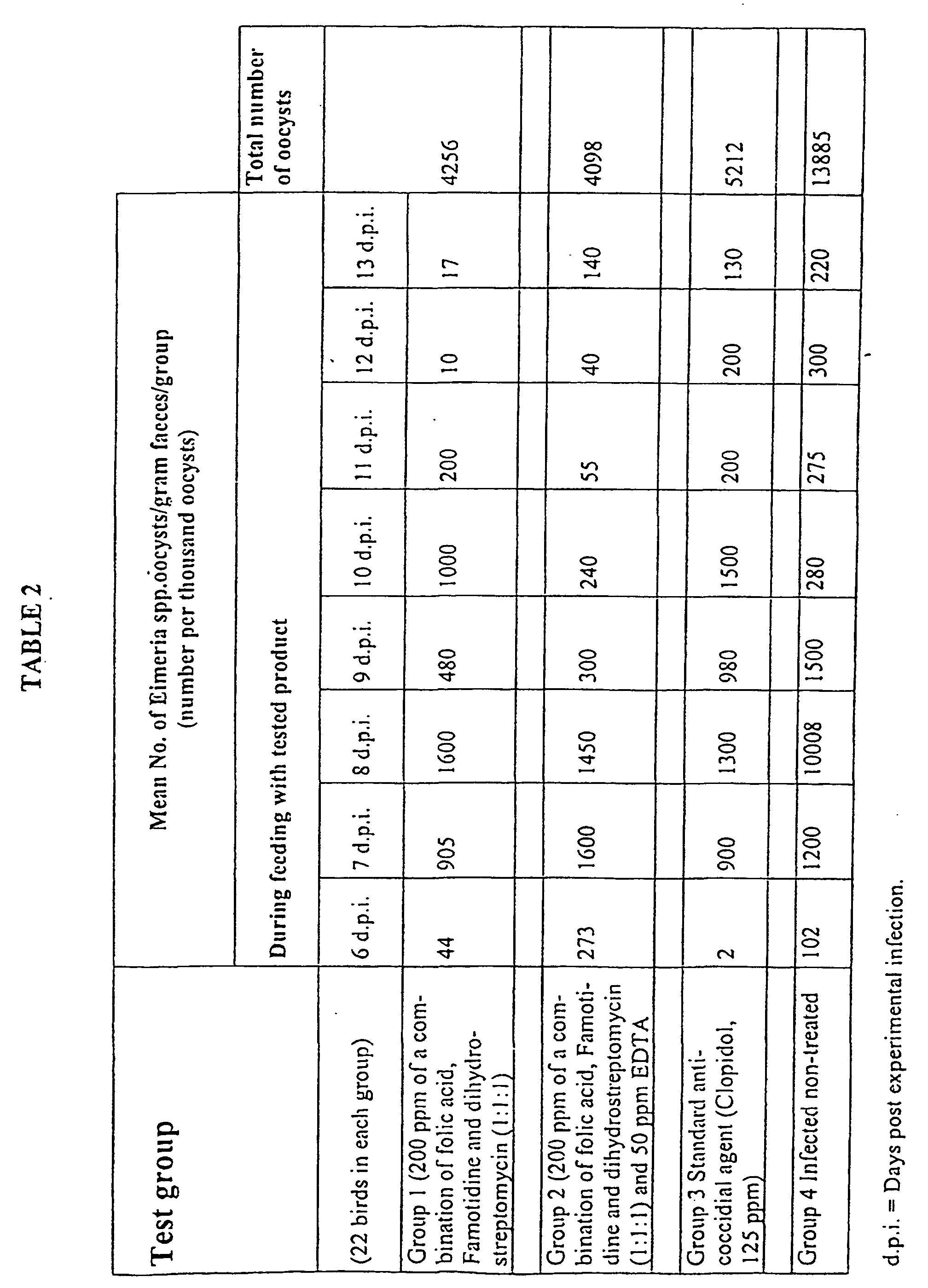

### A3

### Anticoccidiocidal activity

### Materials and Methods

### Chickens

A total of 100 one day old Habbared X breed chickens were used in the test. They were reared on un-medicated feed as one group during the first 3 days. After that they were allocated into 5 groups (20 each) in 5 separate (1 x 1 meter) pens.

### Medication groups:

With the beginning of the day 8 of bird life, the composition to be tested was mixed into the feed of each group and was used for daily feeding. Fresh water was supplied ad-libetum.
The test groups were:

| | |
|---|---|
| Group No. 1 | feed containing 200 ppm dihydrostreptomycin, |
| Group No. 2 | feed containing 100 ppm Famotidine, |
| Group No. 3 | feed containing 100 ppm folic acid, |
| Group No. 4 | feed containing standard anticoccidial agent (Coxistac, 60 ppm), |
| Group No. 5 | infected non-treated group. |

The birds were kept on medicated feed for totally 2 days. On the 3^{rd} day each bird was experimentally infected orally by a mature sporulated mixture of oocysts containing 5 strains, viz. Eimeria acervulina, E. maxima, E. necatrix, E. tenella and E. brunetti.

Fresh fecal droplets were collected daily from the 4^{th} day post experimental infection until day 13 post infection. Mean number of oocyst per gram feaces for each group were calculated using the Mc-Master technique according to Soulsby (1984).
The results appear from Table 3 below.

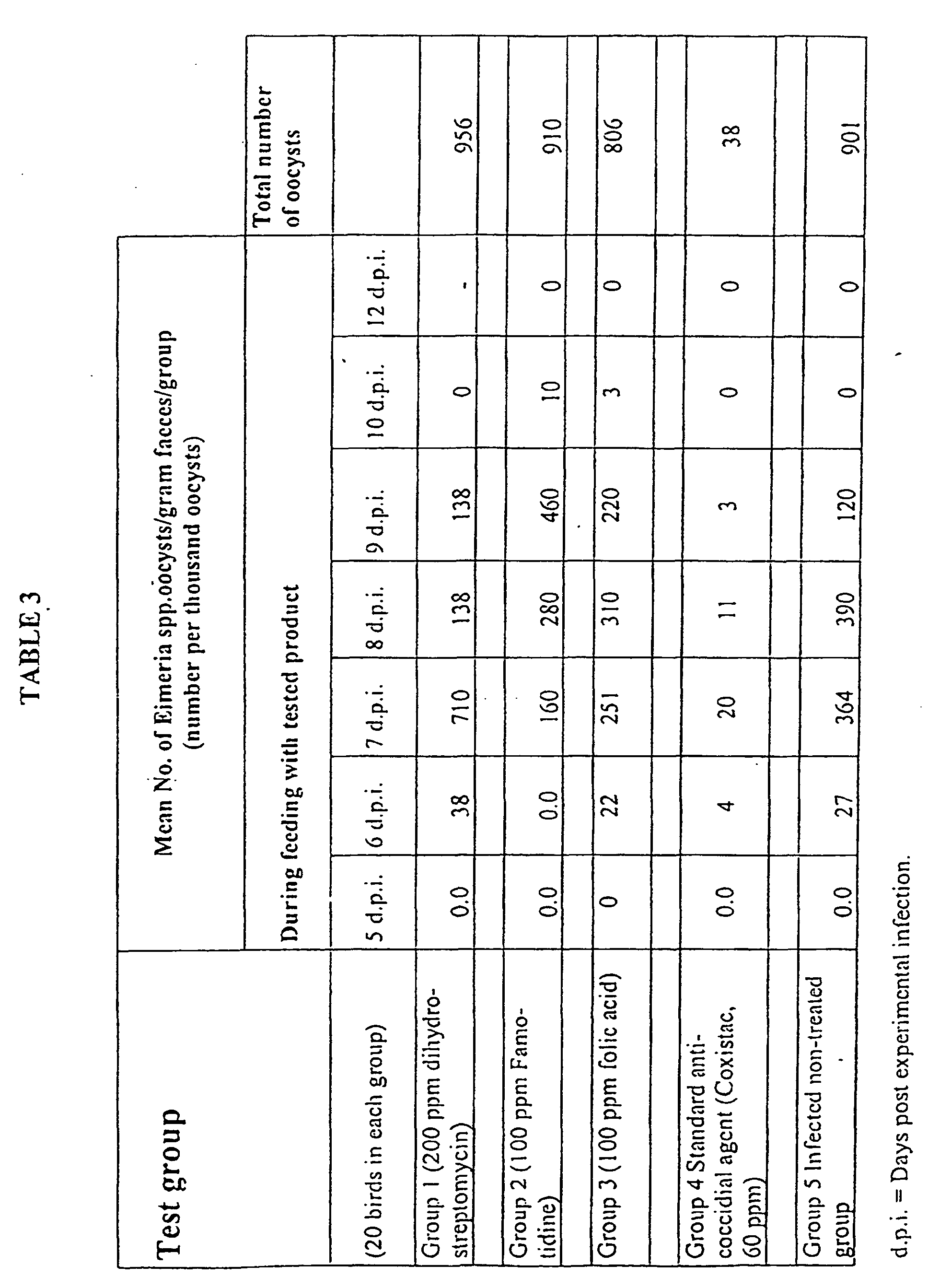

## Claims

1. A composition for use as a medicament comprising a three-component mixture of folic acid, aminoglycosides and antiulcerative agents, said components being present in therapeutically effective amounts, optionally in admixture with therapeutically acceptable carriers.

2. Use of a composition for the manufacture of a medicament for treatment of coccidiosis, said composition comprising a mixture of at least two components selected from the group consisting of folic acid, aminoglycosides and antiulcerative agents.

## Patentansprüche

1. Zusammensetzung für die Verwendung als ein Medikament, mit einer Dreikomponenten-Mischung aus Folsäure, Aminoglykosiden und antiulcerativen Mitteln, wobei die Komponenten in therapeutisch wirksamen Mengen vorhanden sind, wahlweise unter Beimischung von therapeutisch akzeptablen Trägerstoffen.

2. Verwendung einer Zusammensetzung für die Herstellung eines Medikaments für die Behandlung von Kokzidiose, wobei die Zusammensetzung eine Mischung von wenigstens zwei Komponenten, ausgewählt aus der Gruppe bestehend aus Folsäure, Aminoglykosiden und antiulcerativen Mitteln, umfasst.

## Revendications

1. Composition destinée à être utilisée comme médicament comprenant un mélange à trois composants d'acide folique, d'aminoglycosides et d'agents antiulcéreux, lesdits composants étant présents en des quantités thérapeutiquement efficaces, éventuellement en mélange avec des supports thérapeutiquement acceptables.

2. Utilisation d'une composition pour la fabrication d'un médicament pour le traitement de la coccidiose, ladite composition comprenant un mélange d'au moins deux composants choisis dans le groupe consistant en l'acide folique, les aminoglycosides et les agents antiulcéreux.
